# EUROPEAN PATENT SPECIFICATION

(11) **EP 2 575 466 B1**
(45) Date of publication and mention of the grant of the patent: **08.04.2015**
(21) Application number: 11790419.3
(22) Date of filing: 02.06.2011
(51) Int. Cl.: A01N 43/58, A61K 31/50, A61K 31/495, A61P 35/00

(54) **METHODS FOR TREATING METHOTREXATE-RESISTANT DISORDERS WITH 10-PROPARGYL-10-DEAZAAMINOPTERIN**
VERFAHREN ZUR BEHANDLUNG METHOTREXAT-BESTÄNDIGER STÖRUNGEN MIT 10-PROPARGYL-10-DEAZAAMINOPTERIN
MÉTHODES DE TRAITEMENT DE TROUBLES RÉSISTANTS AU MÉTHOTREXATE AVEC 10-PROPARGYL-10-DÉAZAAMINOPTÉRINE

(30) Priority: 02.06.2010 US 350871 P
(43) Date of publication of application: 10.04.2013
(73) Proprietor: Allos Therapeutics, Inc., Westminster, CO 80020 (US)
(72) Inventor: PRONK, Gijsbertus, J., Westminster, US 80020 (US)
(74) Representative: Duffy, Assumpta Dympna
(86) International application number: PCT/US2011/038953
(87) International publication number: WO 2011/153368

(56) References cited:
- WO-A1-98/02163
- US-A- 5 354 751
- US-A1- 2008 058 280
- US-B1- 6 323 205
- WANG E S ET AL: "ACTIVITY OF A NOVEL ANTI-FOLATE (PDX, 10-PROPARGYL 10-DEAZAAMINOPTERIN) AGAINST HUMAN LYMPHOMA IS SUPERIOR TO METHOTREXATE AND CORRELATES WITH TUMOR RFC-1 GENE EXPRESSION", LEUKEMIA AND LYMPHOMA,, vol. 44, no. 6, 1 June 2003 (2003-06-01), pages 1027-1035, XP009053320, ISSN: 1042-8194, DOI: 10.1080/1042819031000077124
- SIROTNAK F M; DEGRAW J I; COLWELL W T; PIPER J R: "A new analogue of 10-deazaaminopterin with markedly enhanced curative effects against human tumor xenografts in mice.", CANCER CHEMOTHERAPY AND PHARMACOLOGY, vol. 42, 1998, - 1998, pages 313-318, XP002698117,
- MOLINA JULIAN R: "Pralatrexate, a dihydrofolate reductase inhibitor for the potential treatment of several malignancies", IDRUGS, CURRENT DRUGS LTD, GB, vol. 11, no. 7, 1 July 2008 (2008-07-01), pages 508-521, XP009170032, ISSN: 1369-7056

## Description

### BACKGROUND OF THE INVENTION

Methotrexate (also, "MTX,") is used as part of combination chemotherapy regimens to treat many kinds of cancers. It is a treatment of many neoplastic disorders including acute lymphoblastic leukemia. Methotrexate is also used as a treatment for some autoimmune diseases, including Myasthenia Gravis, polymyositis, dermatomyositis, inclusion body myositis, ankylosing spondylitis, Crohn's disease, psoriasis, pustular psoriasis, psoriatic arthritis, rheumatoid arthritis, Wegener's granulomatosis, and scleroderma.

10-Propargyl-10-deazaaminopterin (encompassing "10-propargyl-10=dAM," "pralatrexate," "racemic PDX," "(2*S*)-2-[[4-[(1*RS*)-1-[(2,4-diaminopteridin-6-yl)methyl]but-3-ynyl]benzoyl]amino]pentanedioic acid," "(2*RS*)-2-[[4-[(1*RS*)-1-[(2,4-diaminopteridin-6-yl)methyl]but-3-ynyl]benzoyl]anino]pentanedioic acid," and "PDX"), is a compound which has been tested and found useful in the treatment of cancer. 10-propargyl-10-deazaminopterin has been approved by the U.S. Food and Drug Administration (FDA) as a treatment for relapsed and refractory peripheral T-cell lymphoma. 10-propargyl-10-deazaminopterin is also being investigated for use in lymphoma, lung cancer, bladder cancer, and breast cancer.

10-propargyl-10-deazaminopterin was originally disclosed by DeGraw et al., "Synthesis and Antitumor Activity of 10-Propargyl-10-deazaaminopterin," J. Med. Chem. 36: 2228- 2231 (1993) and shown to act as an inhibitor of the enzyme dihydrofolate reductase ("DHFR") and as an inhibitor of growth in the murine L1210 lymphocytic leukemia cell line. In addition, some results were presented for the antitumor properties of the compound using the murine E0771 mammary tumor model.

U.S. Patent No. 6,028,071 and PCT Publication No. WO 1998/02163, disclose that highly purified 10-propargyl-10-deazaminopterin compositions when tested in a xenograft model have efficacy against human tumors. Subsequent studies with 10-propargyl-10-deazaminopterin have shown that it is useful on its own and in combinations with other therapeutic agents. For example, Sirotnak et al., Clinical Cancer Research Vol. 6, 3705-3712 (2000) reports that co-administration of 10-propargyl-10-deazaminopterin and probenecid, an inhibitor of a cMOAT/MRP- like plasma membrane ATPase, greatly enhances the efficacy of 10-propargyl-10-deazaminopterin against human solid tumors. 10-propargyl-10-deazaminopterin and combinations of 10-propargyl-10-deazaminopterin with platinum based chemotherapeutic agents have been shown to be effective against mesothelioma. (Khokar, et al., Clin. Cancer Res. 7: 3199-3205 (2001). Co-administration with gemcitabine (Gem), for treatment of lymphoma, has been disclosed in WO/2005/117892. Combinations of 10-propargyl-10-deazaminopterin with taxols are disclosed to be efficacious in U.S. Patent No. 6,323,205. 10-propargyl-10-deazaminopterin has also shown to be effective for treatment of T-cell lymphoma, see U.S. Patent No. 7,622,470. Other studies have shown a method for assessing sensitivity of a lymphoma to treatment with 10-propargyl-10-deazaminopterin by determining the amount of reduced folate carrier-1 protein (RFC-1) expressed by the sample, wherein a higher level of expressed RFC-1 is indicative of greater sensitivity to 10-propargyl-10-deazaminopterin, disclosed in PCT Publication No. WO 2005/117892.

10-propargyl-10-deazaminopterin is known as an antifolate/antimetabolite. The folate pathway plays a key role in cell growth and proliferation (Appling, 1991; Odin et al, 2003). Folic acid (folate) enters cells via reduced folate carrier 1 (RFC-1), is polyglutamated by folylpolyglutamate synthetase (FPGS), and is reduced to dihydrofolate, which is further converted to tetrahydrofolate (THF) by dihydrofolate reductase (DHFR). The different enzymes and transporters involved in this pathway are targets for an important class of cytotoxic agents: antifolates. Methotrexate was one of the first agents of this class and was first used in the treatment of childhood acute lymphoblastic leukemia (Farber et al, 1948). Since then, methotrexate has been widely used in hematologic and solid cancers and new generations of antifolates have been rationally designed to exploit multiple aspects of the folate pathway (e.g., raltitrexed in colorectal cancer (Cocconi et al, 1998), and pemetrexed in malignant pleural mesotheliomas (Vogelzang et al, 2003) and non-small cell lung carcinomas (NSCLC) (Hanna et al, 2004)). In most tumor cells, RFC-1 mediates internalization of folate analogs. Once inside the cell, these analogs either bind dihydrofolate reductase (DHFR), thereby depleting intracellular reduced folate pools needed for purine and thymidine biosynthesis, or will be metabolized to polyglutamates prior to binding to DHFR. Polyglutamation is catalyzed by folyl-polyglutamate synthetase (FPGS). Folyl-poly glutamate hydrolase (FPGH, also known as gamma-glutamyl hydrolase [GGH]) mediates cleavage and thus subsequent clearance of these intracellular polyglutamated anti-folates. Thymidylate synthase (TS) and glycinamide ribonucleotide formyl transferase (GARFT) are also involved in folate metabolism as "recycling" enzymes (thus directly affecting pools of nucleotides available for DNA synthesis).

Methotrexate is an antifolate. Based on testing of cell lines, it is thought that 10-propargyl-10-deazaminopterin and methotrexate have similar pattern of cytotoxicity, 10-propargyl-10-deazaminopterin being frequently 3- to 19-fold more potent than methotrexate.

Methotrexate resistance occurs usually as a result of mutation or amplification of the DHFR gene. There are three known ways in which a cell may acquire immunity to the effects of this folate antagonist. The concentration of methotrexate in the cell can be diminished by a change in the transport systems that move the drug into and out of the cell. For instance, if there is a reduction in the number of transporters via which methotrexate is taken up by cells, less will be found within the cell. Also, the concentration of the drug in the cell can be regulated by the altered rates of polyglutamation and metabolism. When the drug is more slowly polyglutamated or more rapidly metabolized it can be more easily removed from the cell, decreasing its concentration and activity within the cell. Amplification of the DHFR gene causes an increase in the amount of DHFR present and has been shown to correlate with reduced response to methotrexate treatment. Methotrexate must bind to DHFR to prevent its activity. If a genetic change alters the binding region of DHFR in a way that reduces methotrexate binding, DHFR may continue to activate folates and the effectiveness of the treatment will decrease. All of these outcomes have been implicated in the increased resistance to methotrexate. Resistance to methotrexate may be acquired rapidly, and can lead to treatment failure.

Therefore, methods to overcome acquired methotrexate resistance would be of use.

### SUMMARY OF THE INVENTION

In one embodiment, the instant invention includes a method of treating a methotrexate-resistant disorder in an individual. This method can include administering to the individual an effective amount of a composition comprising 10-propargyl-10-deazaaminopterin or its pharmaceutically acceptable salts.

In another embodiment, the present invention includes a method of treating an individual in need of methotrexate-resistant neoplasia treatment, wherein the method comprises administering to the individual an effective amount of 10-propargyl-10-deazaaminopterin or its pharmaceutically acceptable salts.

In some embodiments, the disorder is a cancer. In other embodiments, the disorder is an inflammatory disorder. In one embodiment, the composition is formulated for intravenous administration. In another, the composition is formulated for oral administration.

### BRIEF DESCRIPTION OF THE DRAWINGS

FIG. 1 shows a synthetic scheme useful in preparing 10-propargyl-10-deazaminopterin;
FIG. 2 shows sensitivity of 10-propargyl-10-deazaaminopterin and other folate inhibitors to 15 cancer cell lines tested;
FIG. 3 shows IC₅₀s for DU-145 cells, naive, 10-propargyl-10-deazaaminopterin -adapted (DU-PDX), and methotrexate-adapted (DU-MTX), when treated with 10-propargyl-10-deazaaminopterin or methotrexate;
FIG. 4 shows IC₅₀s for HEP2 cells, naive, 10-propargyl-10-deazaminopterin-adapted (HEP-PDX), and methotrexate-adapted (HEP-MTX), when treated with 10-propargyl-10-deazaaminopterin or methotrexate;
FIG. 5 shows relative mRNA expression of folate genes in 10-propargyl-10-deazaaminopterin-resistant cell lines;
FIG. 6 shows a Western blot of DHFR protein in DU145 and HEP2 sensitive and DU-PDX, DU-MTX, HEP-PDX and HEP-MTX 10-propargyl-10-deazaaminopterin and metliotrexate-resistant cell lines;
[**0018**] FIG. 7 shows mRNA expression for DHFR in DU145 and HEP2 sensitive and DU-PDX, DU-MTX, HEP-PDX and HEP-MTX 10-propargyl-10-deazaaminopterin and methotrexate-resistant cell lines.

### DETAILED DESCRIPTION OF THE INVENTION

One of the continued problems with therapy in cancer patients is individual differences in response to therapies and genetic changes within tumors that lead to resistance to chemotherapy. Chemotherapy involves the administration of drugs often targeted to rapidly dividing cells. Many chemotherapeutic drugs interfere with DNA replication prior to cell division, including antifolates. Although there have been many advances in chemotherapeutic agents, the genetic instability of cancer cells, especially advanced cancers, leads to a high incidence of drug resistant cancers.

In the present invention, cell lines with acquired resistance to methotrexate and 10-propargyl-10-deazaminopterin were developed from DU145 (prostate) and HEP2 (head & neck) cancer cell lines. Being more than 200-fold resistant to 10-propargyl-10-deazaminopterin than parental cells, DU-PDX and HEP-PDX cells with acquired resistance to 10-propargyl-10-deazaminopterin displayed cross-resistance to methotrexate; DU-MTX and HEP-MTX cells still displayed significant sensitivity to 10-propargyl-10-deazaminopterin.

Different mechanisms of acquired resistance to 10-propargyl-10-deazaminopterin and methotrexate were observed. Acquired resistance to 10-propargyl-10-deazaminopterin was associated with significant decreases in RFC1 mRNA expression in both DU-PDX and HEP-PDX cell lines, in addition, minor increases in MDR1 mRNA expression were also observed. The present invention also shows a slight decrease in FPGS mRNA expression in 10-propargyl-10-deazaminopterin-resistant cells, suggesting a role of polyglutamation in resistance to 10-propargyl-10-deazaminopterin. Studies performed three decades ago discovered that a frequent mechanism of acquired methotrexate resistance is DHFR gene amplification and the consequent enzyme overexpression (reviewed by Assaraf 2007; Chen *et al,* 1995). Hence, upon selection of cultured tumor cell lines in progressively increasing concentrations of methotrexate, acquired antifolate resistance is frequently due to DHFR gene amplification. Indeed, in the present invention, the cell line HEP-MTX, with acquired resistance to methotrexate, displayed a dramatic increase in DHFR protein expression as compared to its parental counterpart, suggesting possible DHFR gene amplification in this model. Such protein increase was not observed in the 10-propargyl-10-deazaminopterin-resistant cell lines. These findings suggest different molecular mechanisms of resistance to methotrexate and 10-propargyl-10-deazaminopterin in these cell lines.

A cancer is "responsive" to a therapeutic agent or there is a "good response" to a treatment if its rate of growth is inhibited as a result of contact with the therapeutic agent, compared to its growth in the absence of contact with the therapeutic agent. Growth of a cancer can be measured in a variety of ways, for instance, the size of a tumor or the expression of tumor markers appropriate for that tumor type may be measured. These criteria define the type of response measured and also the characterization of time to disease progression which is another important measure of a tumor's sensitivity to a therapeutic agent. The quality of being responsive to 10-propargyl-10-deazaminopterin is a variable one, with different cancers exhibiting different levels of "responsiveness" to a given therapeutic agent, under different conditions. Still further, measures of responsiveness can be assessed using additional criteria beyond growth size of a tumor, including patient quality of life, degree of metastases, etc. In addition, clinical prognostic markers and variables can be assessed in applicable situations.

A cancer is "non-responsive" or has a "poor response" to a therapeutic agent such as 10-propargyl-10-deazaminopterin or there is a poor response to a treatment if its rate of growth is not inhibited, or inhibited to a very low degree, as a result of contact with the therapeutic agent when compared to its growth in the absence of contact with the therapeutic agent. As stated above, growth of a cancer can be measured in a variety of ways, for instance, the size of a tumor or the expression of tumor markers appropriate for that tumor type may be measured. The quality of being non-responsive to a therapeutic agent is a highly variable one, with different cancers exhibiting different levels of "non-responsiveness" to a given therapeutic agent, under different conditions. Still further, measures of non-responsiveness can be assessed using additional criteria beyond growth size of a tumor, including patient quality of life, degree of metastases, etc. In addition, clinical prognostic markers and variables can be assessed in applicable situations. Such non responsive or poorly responsive cancers may be non responsive or have been initially responsive, but have acquired resistance. Resistance or non responsiveness may be measured against other, more sensitive cancers or tumor cell lines, or against the same type of cancer or tumor cell lines. These types of cancers may also be referred to as "resistant" cancers to a particular chemotherapeutic, such as, for example, a methotrexate-resistant cancer may be resistant to methotrexate originally, or may have acquired resistance to methotrexate during a course or courses of treatment with methotrexate or another chemotherapeutic. The resistance may be due to a mutation or mutations in proteins in one of the folate pathway proteins, a folate import pathway, or by any other protein relevant for a response to methotrexate.

Accordingly, in one embodiment, the present invention includes a method of treating a methotrexate-resistant disorder in an individual, wherein the method comprises administering to the individual an effective amount of 10-propargyl-10-deazaaminopterin, and its pharmaceutically acceptable salts.

The ability of tumors to acquire resistance to the effects of drugs that previously were toxic to them can result in resistance. Such acquired resistance may result from a chromosomal disruption. Decreased permeability is a common form of intrinsic resistance. Alteration or inactivation of the drug is perhaps the most common mechanism of drug resistance. Drug resistance may also result from a change in the target site on which it acts. Situations in which a disorder, such as a cancer, has acquired resistance to methotrexate can be determined by evaluation of attending physicians or others with skill in the art, and include regression or stasis of the disorder upon treatment, followed by progression of the disorder after a certain period of time of treatment. It has been observed that in many disorders, including cancers, that many disorders manifest acquired resistance to treatment.

Thus in another embodiment the present invention relates to a method of treating a folate pathway dependent disease or malignancy that has acquired resistance to methotrexate during treatment with methotrexate, comprising administering a therapeutically effective amount of a composition comprising 10-propargyl-10-deazaaminopterin or its pharmaceutically acceptable salts.

"Treatment" can mean the use of a therapy to prevent or inhibit further tumor growth, as well as to cause shrinkage of a tumor, and to provide longer survival times. Treatment is also intended to include prevention of metastasis of a tumor. A tumor is "inhibited" or "treated" if at least one symptom (as determined by responsiveness/non-responsiveness, time to progression, or indicators known in the art and described herein) of the cancer or tumor is alleviated, terminated, slowed, minimized, or prevented. Any amelioration of any symptom, physical or otherwise, of a tumor pursuant to treatment using a therapeutic regimen (e.g., 10-propargyl-10-deazaminopterin) as further described herein, is within the scope of the invention. Generally, then, terms "treatment," "treating" and "to treat" as used herein mean to alleviate symptoms, eliminate the causation of a cancer or an inflammatory disorder either on a temporary or a permanent basis, slow the appearance of symptoms and/or progression of the disorder, or prevent disease (i.e. to treat prophylactically). A subject receiving prophylactic treatment is generally a mammal at risk for a cancer or an inflammatory condition due to, for example, genetic predisposition, diet, exposure to disorder-causing agents, exposure to pathogenic agents, and the like.

In one embodiment of the invention, the composition used for the methods of the instant invention can include 10-propargyl-10-deazaminopterin, including "highly purified" 10-propargyl-10-deazaminopterin, and diastereomers of 10-propargyl-10-deazaminopterin. As used in the specification and claims hereof, compositions which are "highly purified" contain 10-propargyl-10-deazaminopterin substantially free of other folic acid derivatives, particularly 10-deazaaminopterin, which can interfere with the antitumor activity of the 10-propargyl-10-deazaminopterin. A composition within the scope of the invention may include carriers or excipients for formulating the 10-propargyl-10-deazaminopterin into a suitable dosage unit form for therapeutic use, as well as additional, non-folate therapeutic agents.

10-propargyl-10-deazaminopterin contains asymmetric centers at the carbon 10 (C10) and carbon 19 (C19) position. In one embodiment, 10-propargyl-10-deazaminopterin includes an approximately 1:1 racemic mixture of the *R*- and *S*-configurations at the C10 chiral center, and ≥ 98.0% of the S-diastereomer at the C19 chiral center. 10-propargyl-10-deazaminopterin includes the C10 diastereomers PDX-10a [*S*-configuration] Chemical name: (2*S*)-2-[[4-[(1*S*)-1-[(2,4-diaminopteridin-6-yl)methyl]but-3-ynyl]benzoyl]amino]pentanedioic acid, and PDX-10b [*R*-configuration] Chemical name: (2*S*)-2-[[4-[(1*R*)-1-[(2,4-diaminopteridin-6-yl)methyl]but-3-ynyl]benzoyl]amino]pentanedioic acid.

10-propargyl-10-deazaminopterin can be synthesized using the method disclosed in Example 7 of DeGraw et al., U.S. Pat. No. 5,354,751, which is directed to manufacturing 10-propargyl-10-deazaminopterin, is incorporated by reference herein in its entirety. 10-propargyl-10-deazaminopterin may also be synthesized by methods presented in U.S. Patent No. 6,028,071, especially in Example 1, which is incorporated by reference herein.

In order to generate diastereomers of 10-propargyl-10-deazaminopterin, 10-propargyl-10-deazaminopterin may be synthesized as taught herein and elsewhere, and either the final product or an earlier intermediate product may be subsequently used as a starting material to separate the C10 diastereomers. Alternately, a chiral synthesis may be employed where substantially pure PDX-10a and/or PDX-10b is produced directly from any of a number of starting materials. Chiral columns to separate enantiomers or diastereomers, known in the art, may be employed to separate the diastereomers of the final 10-propargyl-10-deazaminopterin or an earlier intermediate. Suitable chiral columns for separating the diastereomers include the chiral column CHIRALPAK AD, available from Daicel Chemical Industries Ltd., Japan, using ethanol as the mobile phase.

In some embodiments, the folate pathway dependent disease or disorder is a cancer. In other embodiments, the folate pathway dependent disease or disorder is an inflammatory disorder. In some embodiments, the cancer or inflammatory disorder is a disorder that was initially sensitive to methotrexate, at least to some degree, and then acquired resistance to methotrexate. Methods to determine whether a folate pathway disease or disorder has resistance, either intrinsic or acquired, to methotrexate can be determined by one of skill in the art, and may include such methods as culturing or acquiring relevant cells such as tumor cells from the patient and determining level of expression of various folate pathway enzymes associated with resistance and/or sensitivity to methotrexate. Another such method is by determining and/or monitoring patient response to methotrexate treatment to reveal resistance, either inherent or acquired. The relevant cells may be either genotyped or phenotyped to determine relative expression of markers indicating resistance to methotrexate.

In one embodiment, a phenotypic assay for use in the invention comprises obtaining a tumor explant from a patient, culturing portions of the explant, growing a monolayer of relevant cells from the explant, exposing the monolayer to a drug candidate, and assessing the ability of the drug candidate to alter tumor cell phenotype.

Genotype analysis according to the invention is accomplished by any known method. A preferred method comprises comparing the genotype, or portion thereof, of cells obtained from the patient with genotypes known to be associated with drug resistance generally, or specifically with respect to a therapeutic candidate being evaluated. For example, the existence in patient cells of a polymorphic variant that is known or suspected to confer resistance to a therapeutic candidate would screen that candidate out as a potential therapeutic against those cells. Genetic characteristics of patient cells are determined by methods known in the art (e.g., sequencing, polymorphisms) as set forth below. The impact of a patient's genotype upon drug resistance may be determined by reference to genetic databases or libraries that catalog known mutations or polymorphism related to resistance.

While not being bound to any mechanism, examples of mutations in cells that are associated with resistance or acquired resistance to methotrexate include, but are not limited to, increases in DHFR mRNA and/or protein expression relative to sensitive cells or relative to the cells prior to acquiring resistance.

In some embodiments, the cancer or inflammatory disorder has acquired resistance to methotrexate. Cancers to treat include, for example, prostate cancer, breast cancer, melanoma, lung cancer, and T-cell lymphoma. For T-cell lymphoma, there are a variety of conditions subject to treatment using the diastereomers of the invention, and they include: (a) lymphoblastic lymphomas in which the malignancy occurs in primitive lymphoid progenitors from the thymus; (b) mature or peripheral T cell neoplasms, including T cell prolymphocytic leukemia, T-cell granular lymphocytic leukemia, aggressive NK-cell leukemia, cutaneous T cell lymphoma (mycosis fungoides/Sezary syndrome), anaplastic large cell lymphoma, T-cell type, enteropathy-type T cell lymphoma, Adult T-cell leukemia/lymphoma including those associated with HTLV-1, and angioimmunoblastic T cell lymphoma, and subcutaneous panniculitic T cell lymphoma; and (c) peripheral T cell lymphomas that initially involve a lymph node paracortex and never grow into a true follicular pattern. Other cancers to treat include hematologic malignancies, head and neck cancer, cancer of the gastrointestinal tract, ovarian cancer, and osteosarcoma.

The term "inflammatory disorder" as used herein, refers to any disorder that is either caused by inflammation or whose symptoms include inflammation. By way of example, an inflammatory disorder caused by inflammation may be septic shock, and an inflammatory disorder whose symptoms include inflammation may be rheumatoid arthritis. The inflammatory disorders of the present invention include but are not limited to: cardiovascular disease, rheumatoid arthritis, multiple sclerosis, Crohn's disease, inflammatory bowel disease, systemic lupus erythematosis, polymyositis, septic shock, graft vs. host disease, asthma, rhinitis, psoriasis, and eczema. In one embodiment, an inflammatory disorder to treat includes rheumatoid arthritis and juvenile rheumatoid arthritis.

The term "patient" or "mammal," as used herein, refers to any animal classified as a mammal, including humans, domestic and farm animals, and zoo or companion animals, such as dogs, horses, cats, cattle, etc. Preferably, the mammal is a human.

10-propargyl-10-deazaminopterin for use according to the present invention will typically be administered to the patient in a dose regimen that provides for the most effective treatment (from both efficacy and safety perspectives) for which the patient is being treated, as known in the art. In conducting the treatment method of the present invention, the 10-propargyl-10-deazaminopterin for use in a methotrexate-resistant disorder and/or 10-propargyl-10-deazaminopterin-sensitive cancer according to the present invention can be administered in any effective manner known in the art, such as by oral, topical, intravenous, intra-peritoneal, intramuscular, intra-articular, subcutaneous, intranasal, intra-ocular, vaginal, rectal, intracranial, or intradermal routes, depending upon the type of cancer being treated, and the medical judgment of the prescribing physician as based, e.g., on the results of published clinical studies.

10-propargyl-10-deazaminopterin for use in a methotrexate-resistant disorder and/or 10-propargyl-10-deazaminopterin-sensitive cancer according to the present invention can be formulated as part of a pharmaceutical preparation. The specific dosage form will depend on the method of administration, but may include tablets, capsules, oral liquids, and injectable solutions for oral, intravenous, intramuscular, intracranial, or intraperitoneal administration, and the like. Dosing may be expressed as mg/m². Alternatively, dosing may be expressed as mg/kg body weight by any manner acceptable to one skilled in the art. One method for obtaining an equivalent dosing in mg/kg body weight involves applying the conversion factor 0.025 mg/kg, for an average human, as approximately equivalent to 1 mg/m². According to this calculation, dosing of 150 mg/m² is approximately equivalent to about 3.75 mg/kg.

Appropriate dosing for oncology for treatment of a methotrexate-resistant disorder and/or 10-propargyl-10-deazaminopterin-sensitive cancer includes the following dosage regimes. In one embodiment, these dosages are I.V. For example, doses on the order of 10 to 120 mg/m² of body surface area/day (about 0.25 to 3 mg/kg body weight per day) are appropriate. Dosages of 30 mg/m² (about 0.75 mg/kg) once weekly for 3 weeks followed by a one week rest, 30 mg/m² (about 0.75 mg/kg) once weekly x 6 weeks followed by a one week rest, or gradually increasing doses of 10-propargyl-10-deazaminopterin on the once weekly x 6 week schedule are also suitable. Lower doses may be used as appropriate based on patient tolerance and type of malignancy. Higher doses can be utilized where less frequent administration is used. Thus, in a general sense, dosages of 10 to 275 mg/m² (about 0.25 to about 6.9 mg/kg) are suitably used with various dosing schedules, for example between about 100 to 275 mg/m² (about 2.5 to about 6.87 mg/kg) for biweekly dosages, and between about 10 to 150 mg/m² (about 0.25 to about 3.75 mg/kg), or, more specifically, between about 10 and 60 mg/m² for once weekly dosages.

The determination of suitable dosages using protocols similar to those described in U.S. Pat. No. 6,323,205 is within the skill in the art. In one embodiment, 10-propargyl-10-deazaminopterin for use in a methotrexate-resistant disorder and/or 10-propargyl-10-deazaminopterin-sensitive cancer according to the present invention can be administered in an amount of from about 10 to about 275 mg/m² (about 0.25 to about 6.87 mg/kg) per dose. Methods of the present invention also include administration of 10-propargyl-10-deazaminopterin for use in a methotrexate-resistant disorder and/or 10-propargyl-10-deazaminopterin-sensitive cancer according to the present invention weekly; in a dose of about 10 mg/m² (0.25 mg/kg) or about 30 mg/m² (0.75 mg/kg); in an amount of from about 10 to about 150 mg/m² (about 0.25 to about 3.75 mg/kg) per dose; biweekly; and in a dosage amount of about 100 to about 275 mg/m²(about 2.5 to about 6.9 mg/kg). In one embodiment, 10-propargyl-10-deazaminopterin for use in a methotrexate-resistant disorder and/or 10-propargyl-10-deazaminopterin-sensitive cancer according to the present invention can be administered in an amount of between about 0.25 mg/kg and about 4 mg/kg; between about 0.75 mg/kg and about 3 mg/kg; in an amount between about 1.0 mg/kg and about 2.5 mg/kg; in an amount of about 0.25 mg/kg or about 0.75 mg/kg (or an equivalent amount in body surface area (BSA)).

10-propargyl-10-deazaminopterin may be used in combinations with other cytotoxic and antitumor compounds, including vinca alkaloids such as vinblastine, navelbine, and vindesine, nucleotide analogs such as gemcitabine, 5-fluorouracil, and cytarabine; alkylating agents such as cyclophosphamide or ifosfamide; cisplatin or carboplatin; leucovorin; taxanes such a paclitaxel or docetaxel; anti-CD20 monoclonal antibodies, with or without radioisotopes, and antibiotics such as doxorubicin and mitomycin. Combinations of 10-propargyl-10-deazaminopterin with several of these other antitumor agents or with growth factor inhibitors and anti-angiogenic agents may also be used.

10-propargyl-10-deazaminopterin and other agents may be concurrently administered or utilized in combination as part of a common treatment regimen, in which the 10-propargyl-10-deazaminopterin and the other agent(s) are administered at different times. For example, the other agent may be administered before, immediately afterward or after a period of time (for example 24 hours) relative to the 10-propargyl-10-deazaminopterin administration. Thus, for purposes of this application, the term administering refers generally to concurrent administration or to sequential administration of the drugs and in either order in a parallel treatment regimen with or without a separation in time between the drugs unless otherwise specified.

For treatment of an inflammatory disorder, 10-propargyl-10-deazaminopterin may be given by oral, intramuscular, intravenous, intra-arterial or intrathecal routes. Other routes will occur to those of skill in the art. For treatment of an inflammatory disorder, including, without limitation, psoriasis, rheumatoid arthritis, and/or juvenile rheumatoid arthritis, dosing can include the following. Methods of the present invention for adult rheumatoid arthritis or polyarticular-course Juvenile Rheumatoid Arthritis include oral administration of between about 1 and about 30 mg once weekly; in one embodiment, about 7.5 mg is administered once weekly. Other dosages may include 10 mg/m² given once weekly. Dosages may be adjusted gradually to achieve an optimal response. At higher dosages, such as over 20 mg/m²/wk, or 0.65 to 1.0 mg/kg/wk, better absorption may be achieved by intramuscular or subcutaneous dosages. Appropriate dosing may also include 7.5 mg per week, or divided oral dosages of between about 0.5 and about 10 mg; in one embodiment, dosage may be divided oral dosage of 2.5 mg at 12 hour intervals for three doses given as a course once weekly. Dosing may be continued as long as it is effective, and includes therapy for up to two years and longer.

10-propargyl-10-deazaminopterin is suitably used in combination with folic acid and vitamin B 12 supplementation to reduce the side effects of the treatment. For example, patients may be treated with folic acid (1 mg/m² daily starting 1 week prior to treatment with 10-propargyl-10-deazaminopterin, or alternatively 1 mg perioral (p.o.) daily not based on body surface area (BSA)); and B12 (1 mg/m² monthly, or alternatively given intramuscularly (I.M.) every 8-10 weeks as 1 mg (not based on BSA), or alternatively p.o. daily 1 mg (not based on BSA)).

In one embodiment, 10-propargyl-10-deazaminopterin according to the invention is formulated for oral administration. In another embodiment, 10-propargyl-10-deazaminopterin according to the invention is formulated for intravenous administration.

10-propargyl-10-deazaminopterin can be administered in a wide variety of different dosage forms. For example, 10-propargyl-10-deazaminopterin can preferably be administered orally or parenterally.

10-propargyl-10-deazaminopterin can be administered with various pharmaceutically acceptable inert carriers in the form of tablets, capsules, lozenges, troches, hard candies, powders, sprays, creams, salves, suppositories, jellies, gels, pastes, lotions, ointments, elixirs, syrups, and the like. Administration of such dosage forms can be carried out in single or multiple doses. Carriers include solid diluents or fillers, sterile aqueous media and various non-toxic organic solvents, and others. Oral pharmaceutical compositions can be suitably sweetened and/or flavored. For oral administration of 10-propargyl-10-deazaminopterin, tablets containing one or both of the active agents are combined with any of various excipients such as, for example, micro-crystalline cellulose, sodium citrate, calcium carbonate, dicalcium phosphate and glycine, along with various disintegrants such as starch (and preferably corn, potato or tapioca starch), alginic acid and certain complex silicates, together with granulation binders like polyvinyl pyrrolidone, sucrose, gelatin and acacia. Additionally, lubricating agents such as magnesium stearate, sodium lauryl sulfate and talc are often very useful for tableting purposes. Solid compositions of a similar type may also be employed as fillers in gelatin capsules; preferred materials in this connection also include lactose or milk sugar as well as high molecular weight polyethylene glycols. When aqueous suspensions and/or elixirs are desired for oral administration, 10-propargyl-10-deazaminopterin may be combined with various sweetening or flavoring agents, coloring matter or dyes, and, if so desired, emulsifying and/or suspending agents as well, together with such diluents as water, ethanol, propylene glycol, glycerin and various like combinations thereof. A tablet containing the composition of this invention may be prepared by compression or molding, optionally with one or more accessory ingredients or adjuvants. Compressed tablets may be prepared by compressing, in a suitable machine, the active ingredient in a free-flowing form such as powder or granules, optionally mixed with a binder, lubricant, inert diluent, surface active or dispersing agent. Molded tablets may be made by molding in a suitable machine, a mixture of the powdered compound moistened with an inert liquid diluent. Each tablet preferably contains from about 0.05 mg to about 10 g of the active ingredient and each cachet or capsule preferably containing from about 0.05 mg to about 10 g of the active ingredient; tablets may also suitably contain about 2.5 mg active ingredient per tablet or about 7.5 mg per tablet.

For parenteral administration of 10-propargyl-10-deazaminopterin, solutions may be employed, as well as sterile aqueous solutions comprising the active agent or a corresponding water-soluble salt thereof. Such sterile aqueous solutions are preferably suitably buffered, and are also preferably rendered isotonic, e.g., with sufficient saline or glucose. These particular aqueous solutions are especially suitable for intravenous, intramuscular, subcutaneous and intraperitoneal injection purposes. The oily solutions are suitable for intra-articular, intramuscular and subcutaneous injection purposes. The preparation of all these solutions under sterile conditions is readily accomplished by standard pharmaceutical techniques well known to those skilled in the art.

For veterinary purposes, the active agents can be administered separately or together to animals using any of the forms and by any of the routes described above. In a preferred embodiment, 10-propargyl-10-deazaminopterin is administered in the form of a capsule, bolus, tablet, liquid drench, by injection or as an implant. As an alternative, the 10-propargyl-10-deazaminopterin can be administered with the animal feedstuff, and for this purpose a concentrated feed additive or premix may be prepared for a normal animal feed. Such formulations are prepared in a conventional manner in accordance with standard veterinary practice.

The present invention also includes a method of treating an individual in need of methotrexate-resistant neoplasia treatment, wherein the method comprises administering to the individual an effective amount of 10-propargyl-10-deazaaminopterin, and its pharmaceutically acceptable salts.

The term "pharmaceutically acceptable salts" refers to salts prepared from pharmaceutically acceptable non-toxic bases or acids. When a compound of the present invention is acidic, its corresponding salt can be conveniently prepared from pharmaceutically acceptable non-toxic bases, including inorganic bases and organic bases. Salts derived from such inorganic bases include aluminum, ammonium, calcium, copper (cupric and cuprous), ferric, ferrous, lithium, magnesium, manganese (manganic and manganous), potassium, sodium, zinc and the like salts. Particularly preferred are the ammonium, calcium, magnesium, potassium and sodium salts. In one embodiment, the salt is the hydrochloride salt. Salts derived from pharmaceutically acceptable organic non-toxic bases also include salts of primary, secondary, and tertiary amines, as well as cyclic amines and substituted amines such as naturally occurring and synthesized substituted amines. Other pharmaceutically acceptable organic non-toxic bases from which salts can be formed include ion exchange resins such as, for example, arginine, betaine, caffeine, choline, N',N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylameine, trimethylamine, tripropylamine, tromethamine and the like.

In addition to the common dosage forms set out above, 10-propargyl-10-deazaminopterin (including pharmaceutically acceptable salts, esters, solvates, and polymorphs of each component thereof) may also be administered by controlled release means and/or delivery devices.

Unless otherwise indicated, all numbers expressing quantities of ingredients, dimensions reaction conditions and so forth used in the specification and claims are to be understood as being modified in all instances by the term "about".

In this application and the claims, the use of the singular includes the plural unless specifically stated otherwise. In addition, use of "or" means "and/or" unless stated otherwise. Moreover, the use of the term "including", as well as other forms, such as "includes" and "included", is not limiting. Also, terms such as "element" or "component" encompass both elements and components comprising one unit and elements and components that comprise more than one unit unless specifically stated otherwise.

The invention in part is based on data showing that cells with acquired resistance to methotrexate will retain sensitivity to pralatrexate, whereas, cells that have acquired resistance to pralatrexate will also have acquired resistance to methotrexate. With regard to the predictive genetic factors of pralatrexate sensitivity, two cell lines with acquired resistance to the drug were developed from DU145 (prostate) and HEP2 (head & neck) cancer cell lines. Being more than 200-fold more resistant to pralatrexate than parental cells, DU-PDX and HEP-PDX displayed partial cross-resistance to methotrexate. Pralatrexate acquired resistance was associated with decreased RFC-1 expression and increased MDR1 expression. Fotoohi et al (2009) described antifolate-resistant leukemia lines with mRNA levels of RFC-1 down-regulated more than two-fold in methotrexate-resistant cells, emphasizing the important role of influx transport in antifolate resistance. Similar data were previously obtained by Jansen (1998), Rothen (2004), and Ifergan (2003) using other methotrexate-resistant cellular models. Increased MDR1 expression does not appear to play a role in the observed acquired resistance to pralatrexate since inhibition of MDR1 did not restore sensitivity to pralatrexate. Decreased FPGS activity was shown to be associated with acquired resistance to methotrexate in human leukemia CCRF-CEM cells (Mauritz , 2002). In one study, a slight decrease in FPGS expression was observed in pralatrexate-resistant cells, suggestive of a role for polyglutamation in resistance to pralatrexate. Studies performed three decades ago discovered that another frequent mechanism of acquired methotrexate resistance is DHFR gene amplification and the consequent enzyme overexpression (reviewed by Assaraf 2007; Chen *et al,* 1995). In this study, the cell line HEP-MTX, with acquired resistance to methotrexate, displayed a dramatic increase in DHFR mRNA and protein expression as compared to its parental counterpart. Increased DHFR expression was not observed in the pralatrexate-resistant cell lines. These findings suggest different molecular mechanisms of resistance to methotrexate and pralatrexate in these cell lines.

Thus, the present invention shows that surprisingly, the cell lines that become resistant to methotrexate did not become resistant to pralatrexate and/or retained much more of the original sensitivity to pralatrexate.

Additional objects, advantages, and novel features of the present invention will become apparent to one ordinarily skilled in the art upon examination of the following examples, which are not intended to be limiting. Additionally, each of the various embodiments and aspects of the present invention as delineated hereinabove and as claimed in the claims section below finds experimental support in the following examples.

### EXAMPLES

The following examples are provided for illustrative purposes only and are not intended to limit the scope of the invention.

### Example 1:

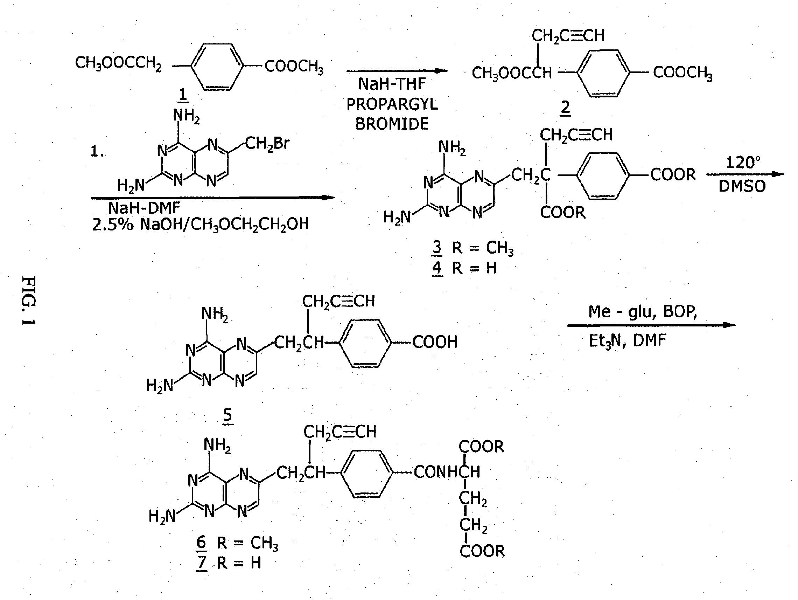

FIG. 1 shows a synthetic scheme useful in preparing 10-propargyl-10-deazaminopterin. A mixture of 60% NaH in oil dispersion (1.06 g, 26.5 mmol) in 18 mL of sieve-dried THF was cooled to 0°C. The cold mixture was treated with a solution of homoterephthalic acid dimethyl ester (5.0 g, 24 mmol. compound 1 in FIG. 1) in dry THF (7 mL), and the mixture was stirred for 1 hour at 0°C. Propargyl bromide (26.4 mmol) was added, and the mixture was stirred at 00°C for an additional 1 hour, and then at room temperature for 16 hours. The resulting mixture was treated with 2.4 mL of 50% acetic acid and then poured into 240 mL of water. The mixture was extracted with ether (2X150 mL). The ether extracts were combined, dried over Na₂SO₄, and concentrated to an orange-yellow oil. Chromatography on silica gel (600 mL of 230-400 mesh) with elution by cyclohexane-EtOAc (8:1) gave the product α-propargylhomoterephthalic acid dimethyl ester (compound 2) as a white solid (4.66) which appeared by TLC (cyclohexane-EtOAc, 3:1) to be homogeneous. Mass spectral data on this product, however, showed it to be a mixture of the desired product 2, and the dipropargylated compound. No starting material 1 was detected. HPLC shows the ratio of mono- to di-propargylated products to be about 3:1. Since the dipropargylated product, unlike compound 1, cannot produce an unwanted coproduct in the next step of the reaction, this material was suitable for conversion to compound 3. Absence of starting compound 1 in the product used to proceed in the synthesis is very important in order to avoid the sequential formation of 10-dAM during the transformations lading to the final product, because complete removal from 10-dAM from 10-propargyl-10-deazaminopterin is very difficult.

A mixture was formed by combining 0.36 g of a 60% NaH (9 mmol) in oil dispersion with 10 mL of dry DMF and cooled to 0-5°C. The cold mixture was treated drop-wise with a solution of the product of the first reaction (compound 2) (2.94 g, 12 mmol) in 10 mL dry DMF and then stirred at 0°C for 30 minutes. After cooling to -25°C, a solution of 2,4,diamino-6-(bromomethyl)-pteridine hydrobromide-0.2 2-propanol (1.00 g, 2.9 mmol) in 10 mL dry DMF was added drop-wise while the temperature was maintained near -25°C. The temperature of the stirred mixture was allowed to rise to -10°C over a period of 2 hours. After an additional 2 hours at -10°C, the temperature was allowed to rise to 20°C, stirring at room temperature was continued for 2 hours longer. The reaction was then adjusted to pH 7 by addition of solid CO₂, After concentration *in vacuo* to remove solvent, the residue was stirred with diethyl ether and the ether insoluble material was collected, washed with water, and dried *in vacuo* to give 1.49 g of a crude product. This crude product was dissolved in CHCl₃-MeOH (10:1) for application to a silica gel column. Elution by the same solvent system afforded 10-propargyl-10-carbomethoxy-4-deoxy-4-a-mino-10-deazapteroic acid methyl ester (compound 3) which was homogenous to TLC in 40% yield (485 mg).

A stirred suspension of compound 3 (400 mg, 0.95 mmol) in 2-methoxyethanol (5 mL) was treated with water (5 mL) and then 10% sodium hydroxide solution (3.9 mL). The mixture was stirred as room temperature for 4 hours, during which time solution occurred. The solution was adjusted to pH 8 with acetic acid and concentrated under high vacuum. The resulting residue was dissolved in 15 mL of water and acidified to pH 5.5-5.8 resulting in formation of a precipitate. The precipitate was collected, washed with water and dried in vacuo to recover 340 mg of compound 4 (91% yield). HPLC analysis indicated a product purity of 90%.

Compound 4 (330 mg) was decarboxylated by heating in 15 mL DMSO at 115-120°C for 10 minutes. A test by HPLC after 10 minutes confirmed that the conversion was essentially complete. DMSO was removed by distillation in vacuo (bath at 40°C). The residue was stirred with 0.5 N NaOH to give a clear solution, Acidification to pH 5.0 with 1N HCl gave 10-propargyl-4-deoxy-4-amino-10-deazapteroic acid (compound 5) as a yellow solid in 70% yield. HPLC indicated product purity at this stage as 90%.

Compound 5 (225 mg, 0.65 mmol) was coupled with dimethyl L-glutamate hydrochloride (137 mg, 0.65 mmol) using BOP reagent (benzotriazole-1-yloxytris(dimethylamino) phosphonium hexafluorophosphate (287 mg, 0.65 mmol, Aldrich Chemical Co.) in DMF (10 mL) containing triethylamine (148 mg, 1.46 mmol). The mixture was stirred for 3 hours at 20-25°C and then evaporated to dryness. The residue was stirred with water, and the water-insoluble crude product was collected and dried *in vacuo.* The crude product (350 mg) was purified by silica gel chromatography with elution by CHCl₃-MeOH (10:1) containing triethylamine (0.25% by volume) to recover 165 mg of 10-propargyl-10-deazaaminopterin dimethyl ester (compound 6, 50% yield) which was homogeneous to TLC (CHCl₃-MeOH 5:1).

Compound 6 (165 mg, 0.326 mmol) was suspended in 10 mL stirred MeOH to which 0.72 mL (0.72 meq) 1N NaOH was added. Stirring at room temperature was continued until solution occurred after a few hours. The solution was kept at 20-25° for 8 hours, then diluted with 10 mL water. Evaporation under reduced pressure removed the methanol, and the concentrated aqueous solution was left at 20-25°C for another 24 hours. HPLC then showed the ester hydrolysis to be complete. The clear aqueous solution was acidified with acetic acid to pH 4.0 to precipitate 10-propargyl-10-deazaaminopterin as a pale yellow solid, The collected, water washed and dried in vacuo product weighed 122 mg (79% yield). Assay by elemental analysis, proton NMR and mass spectroscopy were entirely consistent with the assigned structure. HPLC analysis indicated purity of 98% and established the product to be free of 10-deazaaminopterin.

In this case, the amount of 10-propargyl-10-deazaminopterin (as determined by HPLC peak area) approaches 98%, and the peak corresponding to 10-deazaaminopterin is not detected by the processing software although there is a minor baseline ripple in this area.

### Example 2:

To explore the activity of 10-propargyl-10-deazaminopterin across different solid tumor types, 15 human solid tumor cell lines were investigated for their sensitivity to the cytotoxic activity of 10-propargyl-10-deazaminopterin.

### Materials and Methods: Cell lines

A panel of colon (HT29, HCT116, COLO205, HCC2998), breast (MCF7, MDA-MB-435), lung (HOP62, HOP92), ovarian (OVCAR3, IGROV1), prostate (DU145, PC3), and head and neck (SCC61, HEP2, SQ20B) human cancer cell lines was purchased from the ATCC (Rockville, MD) and National Cancer Institute collections. Cells were grown as monolayers in RPMI medium supplemented with 10% fetal calf serum, 2 mM glutamine, 100 units ml⁻¹ penicillin and 100 µM ml⁻¹ streptomycin.

### Cell cytotoxicity assays

All the data generated was the result of three separate experiments performed in duplicate. Cell viability was determined using the MTT assay, which was carried out as described previously (Hansen, 1989). Briefly, cells were seeded in 96-well plates at a density of 2 x 10³ cells well⁻¹. Cells were incubated for 120 hours and then 0.4 mg ml⁻¹ of MTT dye (3-[4,5-dimethylthiazol-2-yl]-2,5-diphenyltetrazolium bromide was added for 4 hours at 37°C. The monolayer was suspended in 0.1 ml of DMSO and the absorbance at 560 nm was measured using a microplate reader. Positive and negative controls included wells with untreated cells or medium containing MTT with no cells, respectively. The conversion of yellow water-soluble tetrazolium MTT into purple insoluble formazan is catalyzed by mitochondrial dehydrogenases and is used to estimate the number of viable cells. The control value corresponding to untreated cells was taken as 100% and the viability of treated samples was expressed as a percentage of the control. IC₅₀ values were determined as concentrations that reduced cell viability by 50%.

For single agent studies, cells were seeded and allowed to settle for 24 hours prior to treatment with increasing concentrations of 10-propargyl-10-deazaminopterin for 72 h. After incubation, the cells were allowed to recover in compound-free medium for 48 h, prior to determination of growth inhibition using the MTT assay.

### Western blot analysis.

Cells were lysed in buffer containing 50 mM HEPES (pH 7.6), 150 mM NaCl, 1% Triton X-100, 2 mM sodium vanadate, 100 mM NaF, and 0.4 mg.ml⁻¹ phenylmethylsulfonyl fluoride. Equal amounts of protein (20-50 µg/lane) were subjected to SDS-PAGE and transferred to nitrocellulose membranes. Membranes were probed with anti-cleaved PARP, anti-cleaved caspase 3, anti-caspase 9 (Cell Signaling, Saint Quentin Yvelines, France), anti-DHFR (Abcam, France), anti-β-actin (Sigma Aldrich, Saint-Quentin Fallavier, France) specific primary antibodies followed by peroxidase-linked secondary antibodies and visualization by chemiluminescence.

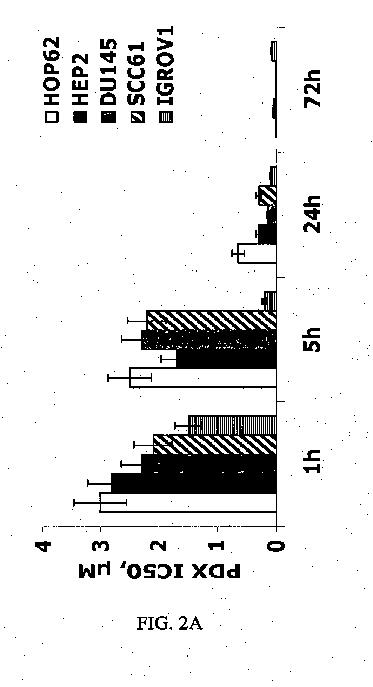

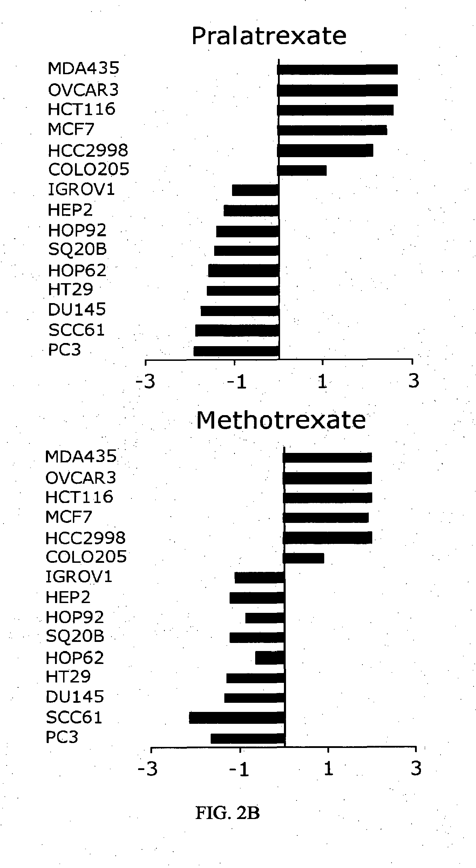

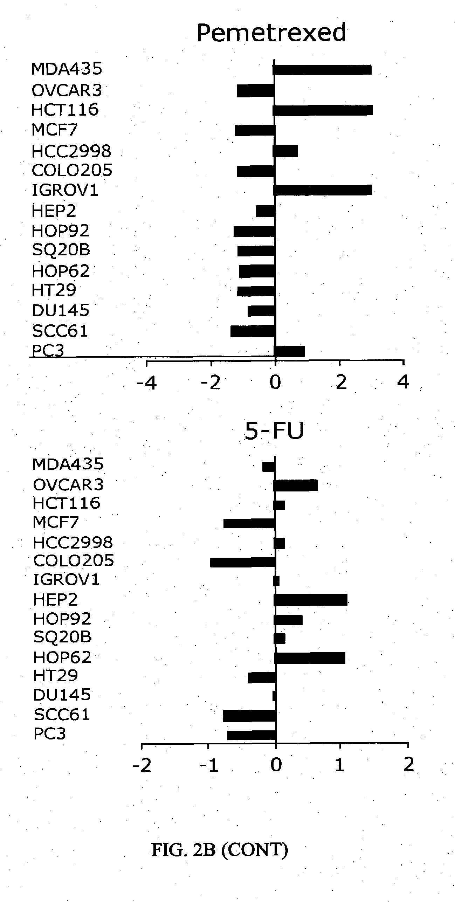

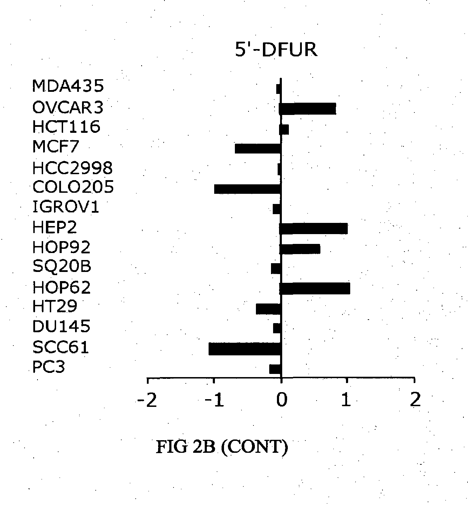

Figure 2 shows the relative sensitivity to 10-propargyl-10-deazaminopterin of the 15 human cancer cell lines tested. Nine of the cell lines were found to be sensitive to the cytotoxic activity of 10-propargyl-10-deazaminopterin (IC₅₀ < 0.1 µM), whereas 6 of the cell lines were found to be relatively resistant (IC₅₀ > 9 µM).

### Single agent antiproliferative effects

The antiproliferative effects of pralatrexate were examined in 15 cancer cell lines as displayed in Table 1. Time course experiments showed that optimal antiproliferative effects were achieved when cells were exposed to pralatrexate for 72h (Figure 1A). Pralatrexate IC₅₀'s ranged from 0.01±0.002 µM for the prostate cancer cell line PC3 to >350 µM for the MDA-MB-435 cell line. Interestingly, two groups of cell lines with more than 100-fold difference in IC₅₀ were observed: One group including PC3, SCC61, DU145, HT29, HOP62, SQ20B, HOP92, HEP2, and IGROV1 cells displayed IC₅₀ <0.1µM, while Colo205, HCC2998, MCF7, HCT116, OVCAR3, and MDA-MB-435 cells showed IC₅₀ values ≥9 µM.

The antiproliferative effects of pralatrexate were compared to those of methotrexate and several commonly used antimetabolites such as pemetrexed, 5-FU, and 5'-DFUR, the active capecitabine metabolite (Figure 1B and Table 1). Pralatrexate IC₅₀s were on average almost 10-fold lower than those observed for methotrexate. The cytotoxicity profiles of these two antifolates were similar with the same distinct groups of sensitive and resistant cell lines. The cytotoxicity profile of pralatrexate was different from that of 5-FU, 5'-DFUR, and pemetrexed, suggesting differences in the metabolism, mechanism of action and/or resistance between pralatrexate and these other antimetabolites. Interestingly, limited cross-sensitivity was observed between pralatrexate and pemetrexed, an antifolate believed to be primarily a thymidylate synthetase (TS) inhibitor.

### Expression of genes involved in folate transport and metabolism

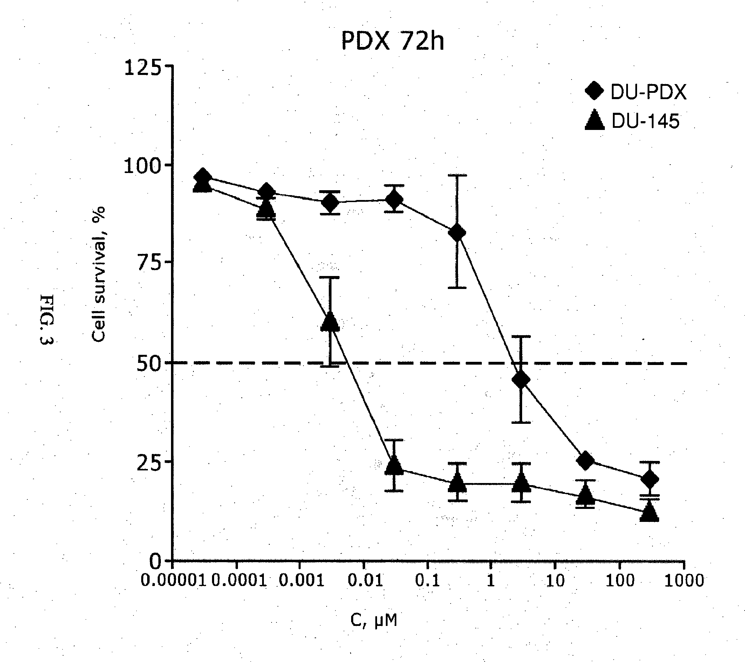

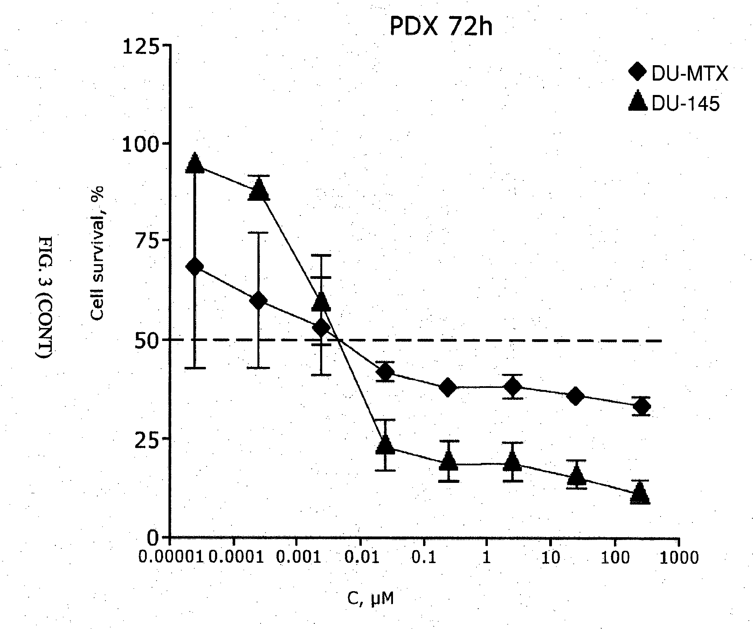

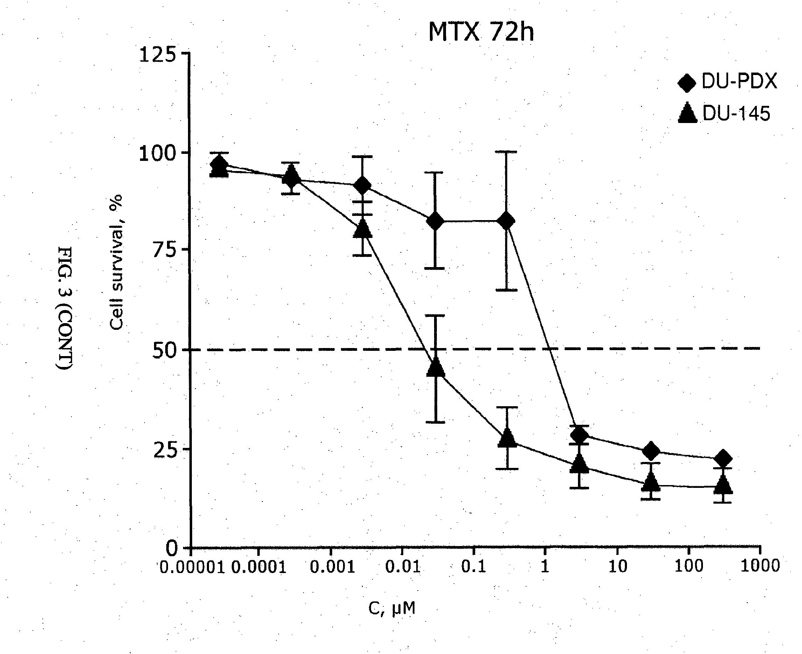

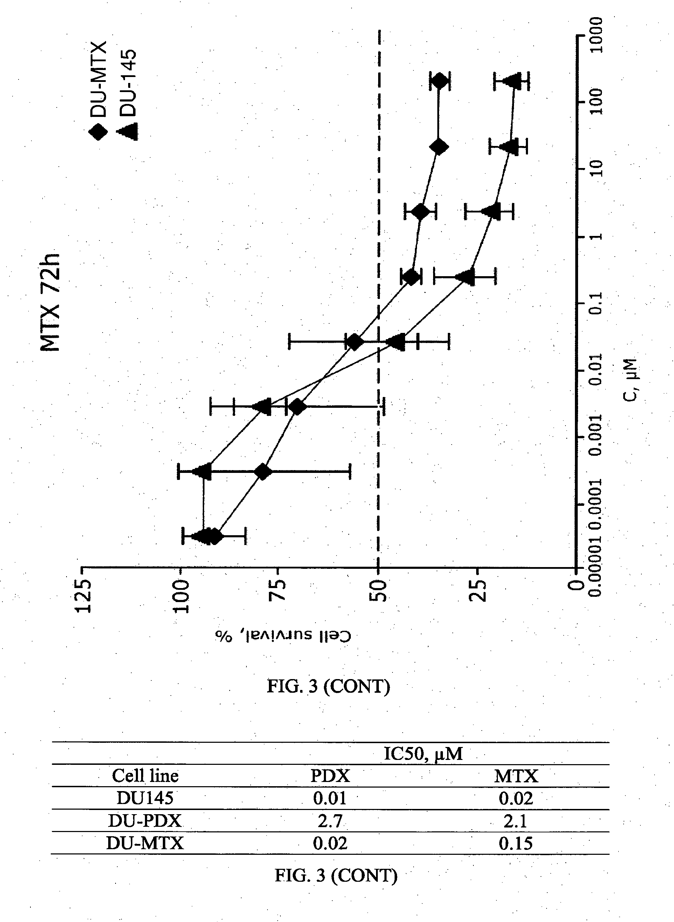

The expression of genes known to be involved in sensitivity to antifolates was analyzed in the panel of cancer cell lines. DHFR, FPGS, TS/TYMS, (thymidylate synthetase), SCL19A1/RFC-1, GARFT (glycinamide ribonucleotide formyl transferase), SLC25A32 (mitochondrial folate transporter/carrier), and ABC transporter B1 (ABCB1 or MDR1) mRNA expression was determined by qRT-PCR (Figure 3A). The cell lines expressed various levels of these folate pathway genes but no significant correlation was found between sensitivity to pralatrexate and mRNA expression of TS, SCL19A1/RFC-1, GARFT, SLC25A32, and MDR1. Pralatrexate-sensitive cells expressed relatively higher levels of DHFR, a target of pralatrexate, than the "resistant" group, but this did not reach statistical significance (p= 0.083, Figure 3A). Pralatrexate-sensitive cells expressed significantly higher levels of FPGS mRNA than resistant cells (t-test, p=0.002). Overall, a trend toward a positive correlation between FPGS mRNA expression and pralatrexate sensitivity (IC₅₀s) was found (R²=0.47, p<0.01), suggesting an important role of polyglutamation in pralatrexate antiproliferative activity.

To determine the potential role of folate transporters in pralatrexate activity, we correlated the IC₅₀ values obtained after 72h drug exposure with the level of mRNA expression of SCL19A1/RFC-1 and SLC25A32 in the nine pralatrexate sensitive cell lines (Figure 3B). Cells that expressed a high level of SCL19A1/RFC-1 and SLC25A32 mRNA displayed higher sensitivity to pralatrexate, suggesting potential roles of SCL19A1/RFC-1 and SLC25A32 in cellular uptake of pralatrexate.

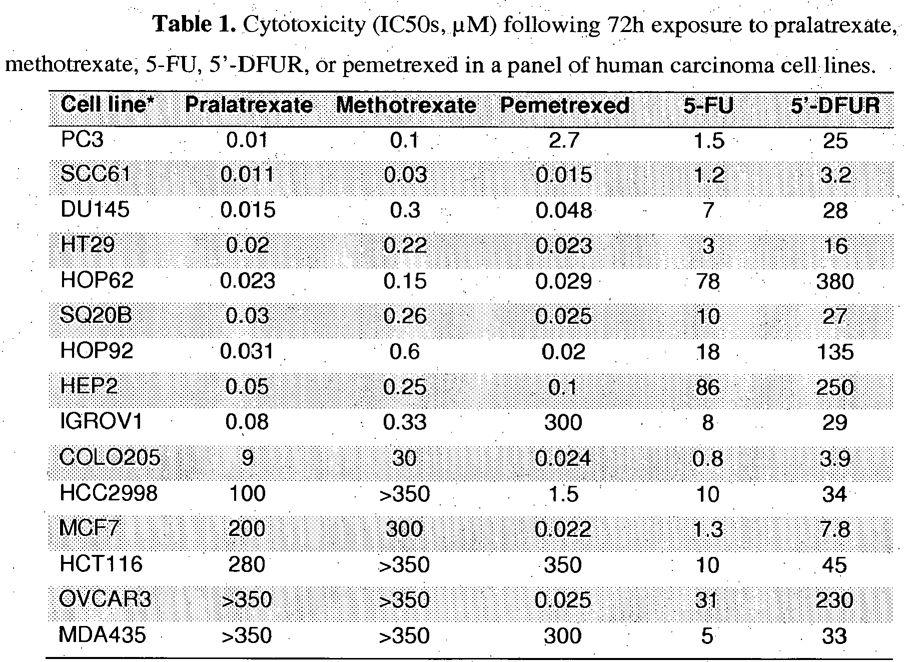

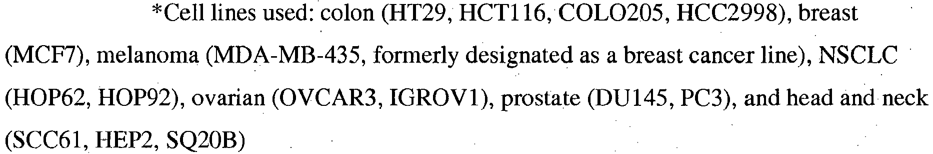

### Example 3:

Development of 10-propargyl-10-deazaminopterin and methotrexate resistant cell lines.

To characterize the predictive factors of 10-propargyl-10-deazaminopterin antiproliferative effects, the cell lines DU-PDX and HEP-PDX were developed from parental DU145 and HEP2 cells, respectively, by exposure to stepwise increasing concentrations of 10-propargyl-10-deazaminopterin over a period of 6 months. Resulting DU-PDX and HEP-PDX cells were at least 200- and 500-fold less sensitive to 10-propargyl-10-deazaminopterin than parental cells. After 5 passages in drug-free medium the resistant cells retained their drug resistance, suggesting stability of these cell lines.

To compare the mechanisms of 10-propargyl-10-deazaminopterin and methotrexate resistance, the cell lines DU-MTX and HEP-MTX were developed from parental DU145 and HEP2 cells by exposure to stepwise increasing concentrations of methotrexate. DU-MTX and HEP-MTX displayed resistance to methotrexate and 10-propargyl-10-deazaminopterin compared to parental cells. However, the activity of 10-propargyl-10-deazaminopterin still remained superior (approximately 10-fold lower IC₅₀) to that of methotrexate in DU-MTX and HEP-MTX cancer cells.

Figure 3 shows IC₅₀ for 10-propargyl-10-deazaminopterin or for methotrexate, for DU145 cells and DU145 cells adapted to either 10-propargyl-10-deazaminopterin or methotrexate. This data shows that for DU-MTX, 10-propargyl-10-deazaminopterin retained significant efficacy; IC₅₀ for 10-propargyl-10-deazaminopterin for the methotrexate adapted DU-145 cell lines was 0.02 µM. In comparison, 10-propargyl-10-deazaminopterin IC₅₀ for the naive DU-145 cells was 0.01 µM. Methotrexate adapted DU-145 cells showed approximately 10 fold increase in IC₅₀ for Methotrexate. In summary, the DU-145 cells that were adapted to 10-propargyl-10-deazaminopterin also became insensitive to methotrexate, whereas the methotrexate adapted DU-145 cells still showed approximately the same sensitivity to 10-propargyl-10-deazaminopterin as the DU-145 naive cells.

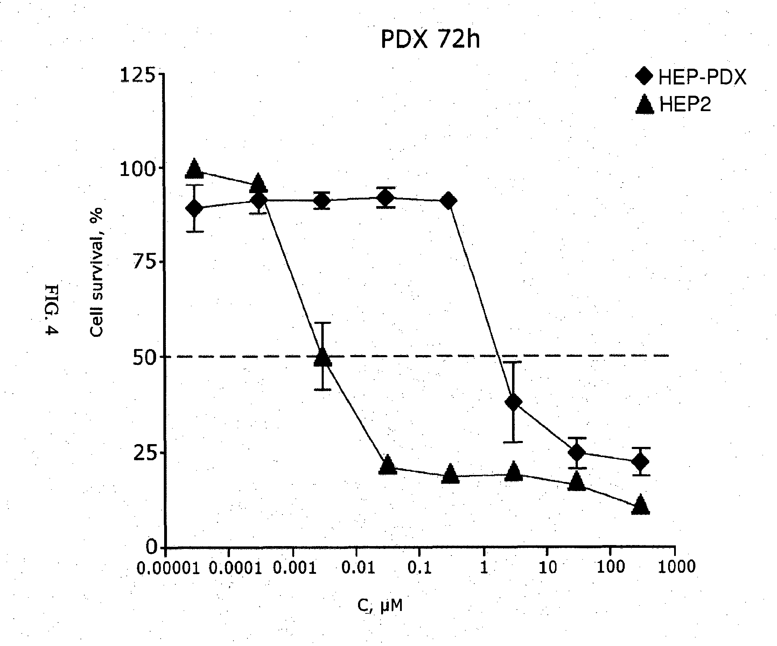

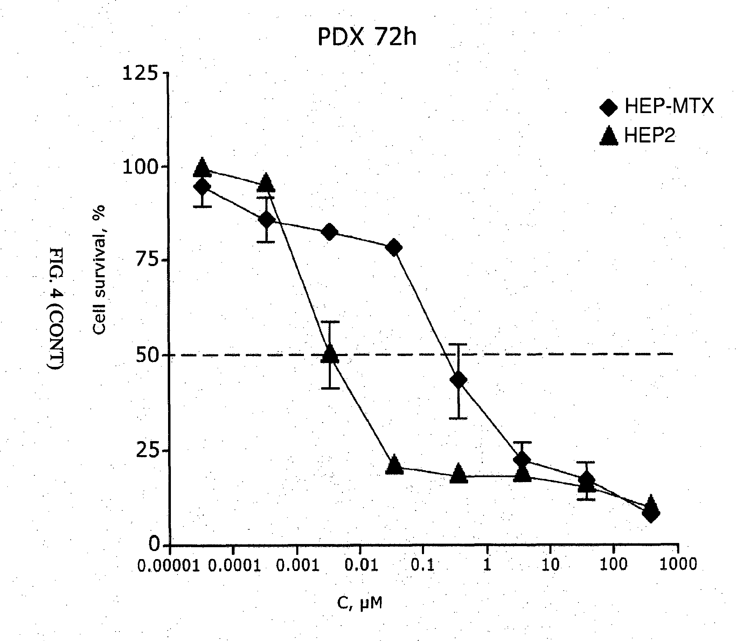

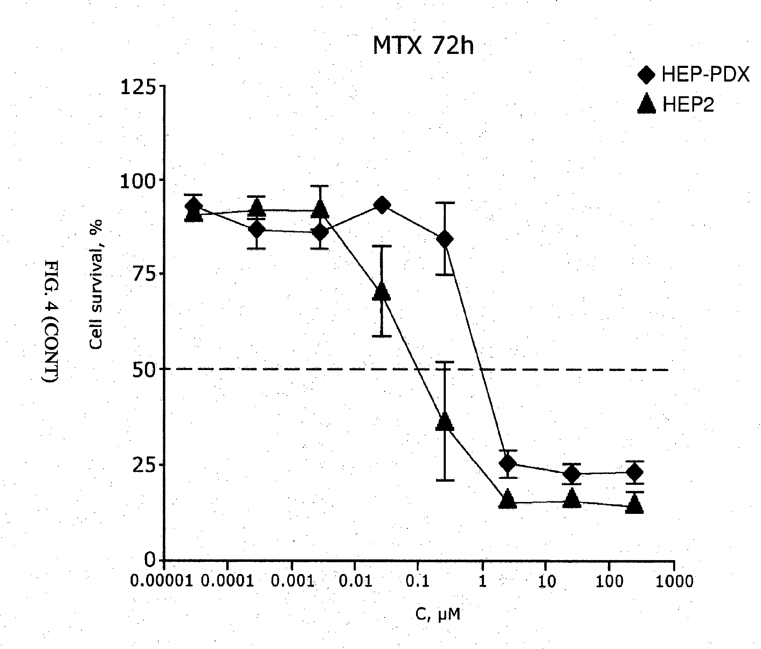

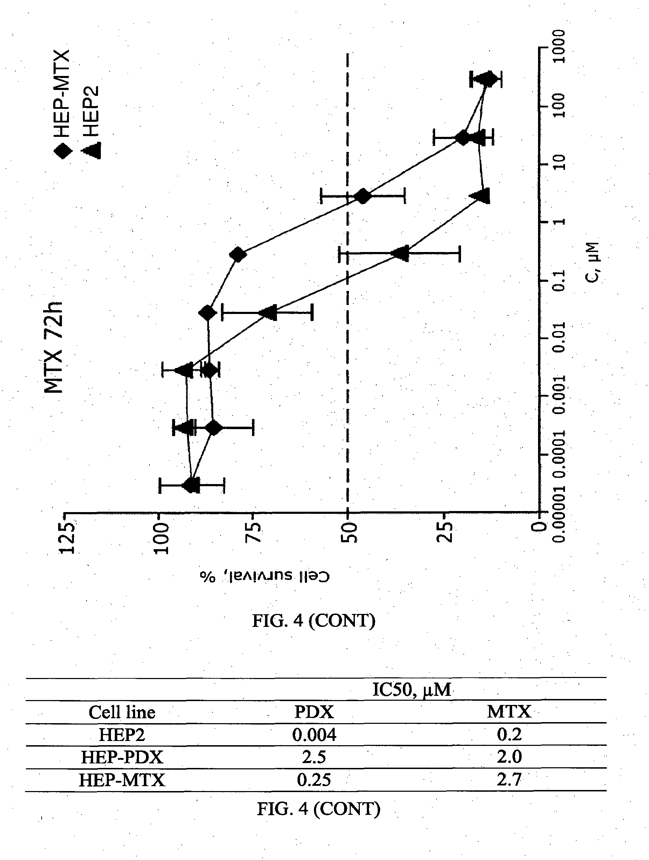

Figure 4 shows IC₅₀ for 10-propargyl-10-deazaminopterin or for methotrexate, for HEP2 cells and HEP2 cells adapted to either 10-propargyl-10-deazaminopterin or methotrexate. This data shows that for HEP-MTX (adapted to methotrexate), 10-propargyl-10-deazaminopterin retained significant efficacy; 10-propargyl-10-deazaminopterin was still ten-fold more effective by IC₅₀ measurement.

Genetic changes associated with acquired 10-propargyl-10-deazaminopterin resistance.

### RT-PCR.

The theoretical and practical aspects of quantitative RT-PCR using the ABI Prism 7900 Sequence Detection System (Perkin-Elmer Applied Biosystems, Foster City, CA, USA) are known to those skilled in the art. Results were expressed as n-fold differences in target gene expression relative to the TBP gene (an endogenous RNA control) and relative to a calibrator (1X sample), consisting of the cell line sample from the tested series that contained the smallest amount of target gene mRNA. Experiments were performed in duplicate.

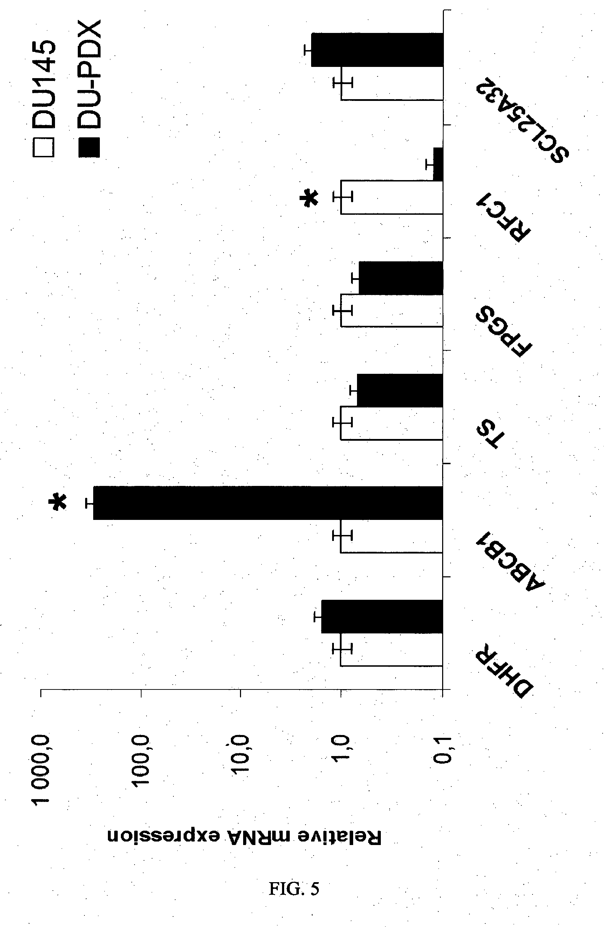

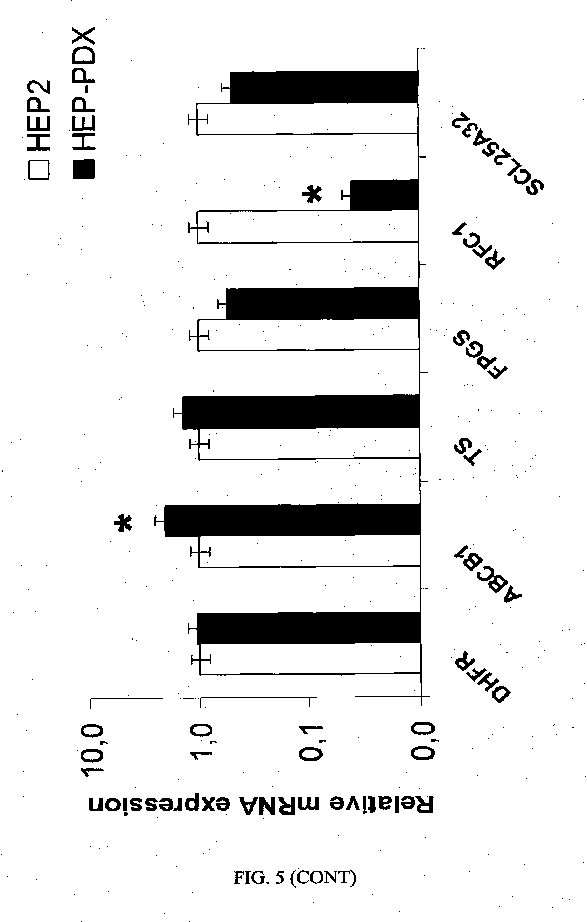

To determine possible mechanisms of anti-folate resistance, we evaluated the mRNA expression of several genes implicated in metabolism of folates including DHFR, TS, FPGS, RFC1/SCL19A1, SLC25A32 and ABCB1/MDR1 in parental and resistant cells. As shown in Figure 5, mRNA expression of DHFR, TS, and SLC25A32 was not significantly changed in 10-propargyl-10-deazaminopterin-resistant cells. A slight decrease in FPGS mRNA expression was observed in DU-PDX and HEP-PDX cells compared with their parental counterparts. In contrast, RFC1/SCL19A1 expression was >10-fold decreased in the two 10-propargyl-10-deazaminopterin-resistant cell lines. mRNA levels of ABCB1/MDR1 was 40- and 2-fold higher in DU-PDX and HEP-PDX, respectively, compared with DU145 and HEP2. These data suggest an important role of transporters in 10-propargyl-10-deazaminopterin antiproliferative activity and acquired resistance. Verapamil, a calcium channel blocker, reverses resistance by functioning as a competitive substrate of MDR1, regardless of its innate pharmacological function. Various clinical studies also showed that drugs such as verapamil could reverse resistance to anticancer drugs. To study the role of MDR1 in 10-propargyl-10-deazaminopterin resistance, DU-PDX and HEP-PDX cells were incubated with 30µM verapamil and 3µM cyclosporin A concomitantly with 10-propargyl-10-deazaminopterin for 72 hours. No changes were observed in 10-propargyl-10-deazaminopterin cytotoxicity with and without verapamil and cyclosporine A, suggesting no significant role of MDR1 overexpression in acquired resistance in these cell lines (results not shown).

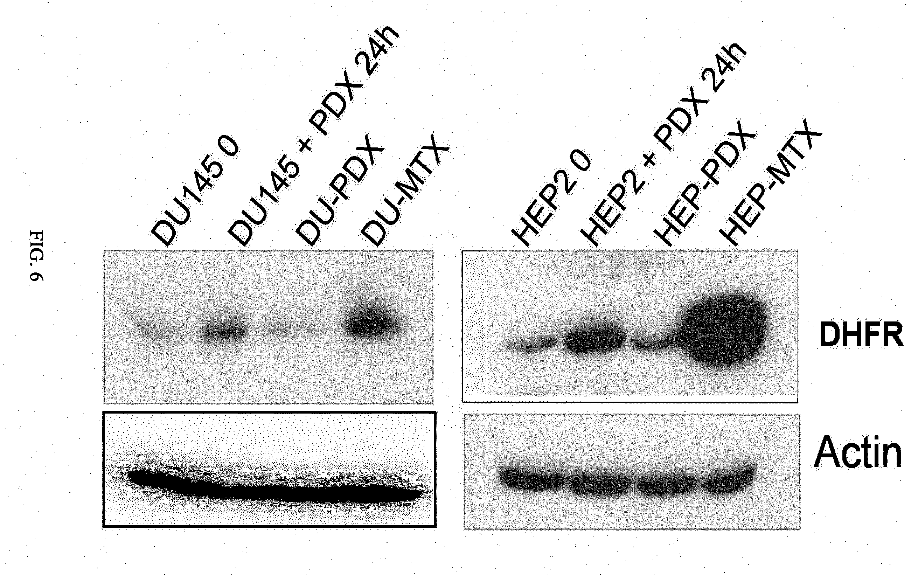

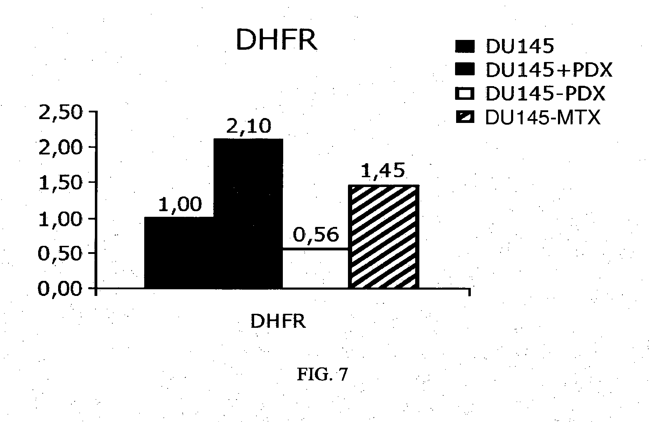

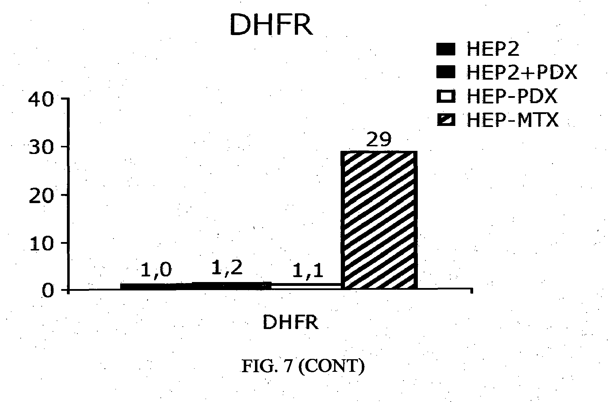

Analysis of expression of DHFR, a target of 10-propargyl-10-deazaminopterin and methotrexate, showed significant increases in protein in HEP-MTX cells compared with parental HEP2 cells suggesting possible gene amplification (Figure 6) and significant increases in mRNA (Figure 7). DHFR protein expression was slightly increased after short (24 hour) exposure to 10-propargyl-10-deazaminopterin, but not after prolonged (6 months) exposure to 10-propargyl-10-deazaminopterin, suggesting that the molecular mechanism of acquired resistance to 10-propargyl-10-deazaminopterin in HEP-PDX cells is different from methotrexate resistance in HEP-MTX cells.

To evaluate the cross-resistance of pralatrexate-resistant cells to other drugs, DU145, DU-PDX, HEP2 and HEP-PDX cells were exposed to pemetrexed and 5-FU for 72h. No significant difference between parental and PDX-resistant cells was observed for 5-FU cytotoxicity. Pemetrexed exposure for 72 hr was only slightly less cytotoxic in DU-PDX and HEP-PDX cells compared to their parental counterparts (data not shown). These data suggest that acquired resistance to pralatrexate may not translate into resistance to pemetrexed and 5-FU, possibly due to the differences in mechanism of action of these compounds.

Pralatrexate is an antifolate with high affinity for the reduced folate carrier 1 (RFC-1) protein and folylpolyglutamate synthetase (FPGS), resulting in extensive internalization and accumulation within tumor cells. Pralatrexate is currently being investigated as a single agent and in combinations in various malignancies. In order to guide further clinical development, molecular correlates of sensitivity to pralatrexate and preclinical data on combination treatments are needed.

Pralatrexate displayed potent antiproliferative activity (IC₅₀ <0.1 µM) in nine out of the 15 human solid tumor cell lines. Two distinct groups of cell lines were identified with >100-fold difference in pralatrexate IC₅₀ values: sensitive and relatively resistant cell lines. The *in vitro* antiproliferative effects of pralatrexate in terms of IC₅₀ values were on average almost 10-fold better than those observed with methotrexate. When comparing the cytotoxic activity of these two similar antifolates to other antimetabolites including 5-FU, 5'-DFUR and pemetrexed, pralatrexate appears to retain activity in several cells that were poorly sensitive to 5-FU and 5'-DFUR, such as NSCLC HOP62 and HOP92 cell lines. Similarly, the sensitivity profile for pemetrexed was different from that for pralatrexate, which may be explained the differences in molecular mechanism of action of these compounds.

In summary, acquired resistance to 10-propargyl-10-deazaminopterin was associated with decreased RFC-1 and increased MDR1 expression in DU-PDX and HEP-PDX cell lines. Pharmacologic inhibition of MDR1 did not change 10-propargyl-10-deazaminopterin resistance in these models, suggesting a limited role of MDR1 in the observed resistance. No change in DHFR mRNA expression was found in 10-propargyl-10-deazaminopterin-resistant cells. In contrast, significant increases in DHFR mRNA and protein expression were seen in HEP-MTX cells. Differences in the mechanisms of acquired resistance for 10-propargyl-10-deazaminopterin and methotrexate were observed; this data suggests further development of 10-propargyl-10-deazaminopterin in methotrexate sensitive cancers as well as in settings of acquired methotrexate resistance.

## Claims

1. A composition comprising an effective amount of 10-propargyl-10-deazaaminopterin or its pharmaceutically acceptable salts for use in treating a methotrexate-resistant disorder in an individual having a methotrexate-resistant disorder, wherein the methotrexate-resistant disorder is an acquired methotrexate-resistant disorder.

2. A composition for use according to claim 1, wherein the disorder is a cancer.

3. A composition for use according to claim 2, wherein the cancer is prostate cancer, T-cell lymphoma, breast cancer, lung cancer, hematologic malignancies, head and neck cancer, cancer of the gastrointestinal tract, ovarian cancer, and osteosarcoma.

4. A composition for use according to claim 1, wherein the disorder is an inflammatory disorder.

5. A composition for use according to claim 4, wherein the inflammatory disorder is rheumatoid arthritis.

6. A composition for use according to claim 1, wherein the disorder is a neoplasia.

7. A composition for use according to claim 6, wherein the neoplasia is prostate cancer, T-cell lymphoma, breast cancer, lung cancer, hematologic malignancies, head and neck cancer, cancer of the gastrointestinal tract, ovarian cancer, and osteosarcoma.

8. A composition for use according to any preceding claim, wherein said composition is formulated for oral administration.

9. A composition for use according to any one of claims 1-7, wherein said composition is formulated for intravenous administration.

## Patentansprüche

1. Zusammensetzung, die eine wirksame Menge an 10-Propargyl-10-deazaaminopterin oder dessen pharmazeutisch verträglichen Salzen umfasst, für die Verwendung bei der Behandlung einer Methotrexat-resistenten Erkrankung bei einem Individuum, das eine Methotrexat-resistente Erkrankung hat, worin die Methotrexat-resistente Erkrankung eine erworbene Methotrexat-resistente Erkrankung ist.

2. Zusammensetzung für die Verwendung nach Anspruch 1, worin die Erkrankung ein Krebs ist.

3. Zusammensetzung für die Verwendung nach Anspruch 2, worin der Krebs Prostatakrebs, T-Zell-Lymphom, Brustkrebs, Lungenkrebs, hämatologische Malignome, Kopf- und Halskrebs, Krebs des Magen-Darm-Trakts, Eierstockkrebs und Osteosarkom ist.

4. Zusammensetzung für die Verwendung nach Anspruch 1, worin die Erkrankung eine entzündliche Erkrankung ist.

5. Zusammensetzung für die Verwendung nach Anspruch 4, worin die entzündliche Erkrankung rheumatoide Arthritis ist.

6. Zusammensetzung für die Verwendung nach Anspruch 1, worin die Erkrankung eine Neoplasie ist.

7. Zusammensetzung für die Verwendung nach Anspruch 6, worin die Neoplasie Prostatakrebs, T-Zell-Lymphom, Brustkrebs, Lungenkrebs, hämatologische Malignome, Kopf- und Halskrebs, Krebs des Magen-Darm-Trakts, Eierstockkrebs und Osteosarkom ist.

8. Zusammensetzung für die Verwendung nach einem vorstehenden Anspruch, worin die genannte Zusammensetzung für eine orale Verabreichung formuliert ist.

9. Zusammensetzung für die Verwendung nach einem der Ansprüche 1-7, worin die genannte Zusammensetzung für eine intravenöse Verabreichung formuliert ist.

## Revendications

1. Composition comprenant une quantité efficace de 10-propargyl-10-déazaaminoptérine ou ses sels pharmaceutiquement acceptables, pour une utilisation dans le traitement d'un trouble résistant au méthotréxate chez un individu présentant un trouble résistant au méthotréxate, où le trouble résistant au méthotréxate est un trouble résistant au méthotréxate acquis.

2. Composition pour une utilisation selon la revendication 1, où le trouble est le cancer.

3. Composition pour une utilisation selon la revendication 2, où le cancer est le cancer de la prostate, le lymphome à cellules T, le cancer du sein, le cancer du poumon, les malignités hématologiques, le cancer de la tête et du cou, le cancer du tractus gastrointestinal, le cancer de l'ovaire et l'ostéosarcome.

4. Composition pour une utilisation selon la revendication 1, où le trouble est un trouble inflammatoire.

5. Composition pour une utilisation selon la revendication 4, où le trouble inflammatoire est la polyarthrite rhumatoïde.

6. Composition pour une utilisation selon la revendication 1, où le trouble est une néoplasie.

7. Composition pour une utilisation selon la revendication 6, où la néoplasie est le cancer de la prostate, le lymphome à cellules T, le cancer du sein, le cancer du poumon, les malignités hématologiques, le cancer de la tête et du cou, le cancer du tractus gastrointestinal, le cancer de l'ovaire et l'ostéosarcome.

8. Composition pour une utilisation selon l'une quelconque des revendications précédentes, où ladite composition est formulée pour une administration orale.

9. Composition pour une utilisation selon l'une quelconque des revendications 1-7, où ladite composition est formulée pour une administration intraveineuse.
